# EUROPEAN PATENT SPECIFICATION

(11) **EP 2 384 727 B1**
(45) Date of publication and mention of the grant of the patent: **17.06.2015**
(21) Application number: 10191247.5
(22) Date of filing: 15.11.2010
(51) Int. Cl.: A61F 9/008, A61B 18/20

(54) **Ophthalmic laser treatment system**
Ophtalmisches Laserbehandlungssystem
Système de traitement laser ophtalmologique

(30) Priority: 06.05.2010 EP 10162195
(43) Date of publication of application: 09.11.2011
(73) Proprietor: Meridian AG, 3608 Thun (CH)
(72) Inventor: Widmer, Markus, Dr., 3600 Thun (CH)
(74) Representative: Patentbüro Paul Rosenich AG

(56) References cited:
- WO-A1-2004/027487
- US-B1- 6 381 255

## Description

The invention relates to an ophthalmic laser treatment system comprising a first beam path incorporating a laser module for producing a beam of short pulses of radiation with high energy density at a first wavelength and along a beam direction, and a beam attenuator means downstream said laser module; a second beam path; a third beam path; an optical switching means allowing alternatively directing the beam from the first beam path into the second beam path or into the third beam path; said second beam path incorporating a beam expander for expanding said beam of short pulses at said first wavelength; said third beam path incorporating downstream of the optical switching means a frequency doubling module; and means for directing the beam of the second and third beam path into the eye of a patient.

In prior art several devices are known which can be used for treating both Glaucoma (abnormal intra-ocular pressure) and secondary cataracts. A promising method to treat Glaucoma is known as selective laser trabeculoplasty (SLT). The aim of this method is to create small openings within the trabecular meshwork. In contrast to argon laser trabeculoplasty (ALT), SLT utilizes a pulsed laser (the pulse duration is short compared to thermal effects) so there is minimal heat transfer to surrounding tissue. This technique is described in US 5,549,596 A and uses a frequency doubled Nd : YAG laser for the SLT procedure.

The preferred ophthalmic treatment for treating secondary cataracts is secondary cataract surgery. The most effective laser for secondary cataract surgery is a Nd : YAG laser operating at a wavelength of 1064nm. These lasers are typically referred to as photodisruptors as they act by non-thermal mechanisms to cut tissue. The main advantage of such ophthalmic laser systems consists in the fact, that only one laser system is necessary to perform the procedures for treating these two most common eye problems of Glaucoma and secondary cataracts.

For the invention at hand, WO 2004/027487 A1 is regarded as the closest prior art. WO 2004/027487 A1 discloses an ophthalmic laser system for generating a first beam at a wavelength suitable for performing selective laser trabeculoplasty (SLT) and selectively generating a second beam at a wavelength suitable for performing secondary cataract surgery procedures. Both beam paths are feed by the same laser source. Means for selecting causes the first beam or the second beam to choose different paths to the eye of the patient according to the wavelength desired. A frequency doubling module is provided in the beam path for performing SLT, in order to change the wavelength of the laser beam towards the visible range.

A beam attenuator is provided at a position just before the beam path of the laser module is splittable into two different beam paths. The attenuator consists of a half wave plate in co-operation with a polarising plate. The orientation of the half wave plate with respect to the polarising plate determines the amount of the pulsed beam that is passed through the polarizing plate into the photodisruptor optical system for treating secondary cataracts. This attenuator appears to be only suitable for regulation of the beam intensity for the photodisruptor mode for treating secondary cataracts.

If the SLT mode is required, the half wave plate is oriented so that all the beam is reflected from the polarising plate to the beam path of the SLT optical system. A separate beam attenuator has to be provided in the SLT beam path in order to regulate the beam intensity for trabeculoplasty. The beam directed to the SLT beam path passes, in the following order, the frequency doubling module, the beam attenuator, an energy monitoring system and finally a beam shaping module.

The configuration of such a ophthalmic laser system is relatively complicated and requires a lot of space for the arrangement of its elements. Further the efficiency of the original laser beam is not optimally used.

US 6,066,127 A (in the same patent family as DE 19 844 719 A1) discloses a laser treatment apparatus which performs a medical or surgical treatment having two parallel beam paths which are coupled to the same laser source. The first beam path has a Q-switch which emits light oscillated by the solid-state laser medium as a pulse wave laser beam, and a second beam path which emits the light oscillated by the solid-state laser medium as a continuous wave laser beam. The laser beam is switched to one of the two optical paths. With this apparatus, plural oscillation modes and wavelengths can be switched over in accordance with the treatment object. At the end of the beam path having the Q-switch a polarizing plate is arranged for attenuating the output power of the pulsed laser beam. The beam path producing a cw laser beam has a nonlinear crystal for generating second harmonic waves and a mirror reflecting the fundamental wavelength and allowing several percentage of the second harmonic to pass. There are no further attenuators or beam shapers for spatially shaping the beams within the two beam paths or between the laser source an the two beam paths. Thus, the controllability and also the efficiency of the beam conversion leave a lot to be desired. However, the main difference with respect to the WO 2004/027487 A1 consists in the fact that only one of the beam paths is Q-switched. The frequency doubled cw pulse is not suited for treating Glaucoma via the trabecular meshwork.

US 5,066,291 A discloses a conversion system for medical applications wherein laser radiation at a predetermined wavelength from a single laser device is converted for simultaneous application of laser light energy at a plurality of predetermined wavelengths. This system has only one beam path. In an embodiment the system has a removable frequency doubling module. Due to the fact that this system has only one beam path the concept is completely different from the closest prior art, WO 2004/027487 A1.

US 4,309,998 A discloses an apparatus for ophthalmological surgery and a method for producing a short pulse ophthalmic laser beam with optional frequency doubling. However only one beam path is used for the two options.

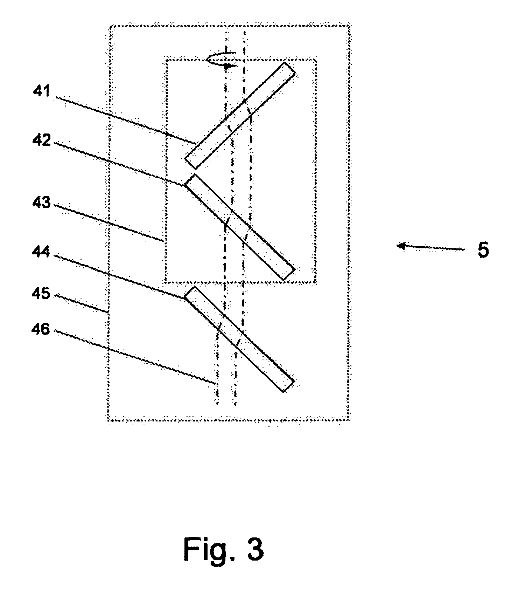

US 4,791,927 A discloses a dual-wavelength laser system for both cutting and coagulating tissue, having a laser source, which wavelength is such that the laser is effective for cauterizing bleeding blood vessels through photocoagulation. The system further comprises means for selectively doubling the wavelength of the original laser beam to a second wavelength beam (e.g. frequency doubler), said second wavelength being in the range of 250-400 nm such being effective for cutting tissue by photoablation. Beam delivery means are provided for directing at least one of the respectively selected laser beams onto a target. In Fig. 3 an embodiment with two parallel beam paths is shown. Each beam path has separate intensity control means. Comparable to documents described above the controllability of the single beams as well as the efficiency of the beam conversion when frequency doubled are not optimized. However the main difference to WO 2004/027487 A1 consists in the fact that this system is not suited for ophthalmic treatments.

The object of the invention is to provide an ophthalmic laser system which on the one hand is most efficient in the frequency conversion of the laser beam and at the same time producing an optimally shaped beam for trabeculoplasty and on the other hand allows to attenuate a beam used for trabeculoplasty as well as a beam used for cataract surgery in a reliable, cost-effective and space-saving manner.

These objects are achieved with an ophthalmic laser system mentioned above and according to claim 1, wherein the optical switching means is arranged downstream of said beam attenuator means, the third beam path comprises a beam shaping module upstream of said frequency doubling module; any beam attenuation in the third beam path is solely controllable by said beam attenuator means in the first beam path, and the frequency doubling module comprises a temperature stabilizing component for stabilizing the temperature of a frequency doubling element of the frequency doubling module. The temperature stabilizing component can be a heating element. Advantageously the temperature stabilizing component is provided as a Peltier element, for preferably stabilizing the temperature of the frequency doubling element between 20°C and 40°C, most preferably between 27°C and 33°C. A temperature around 30°C has been verified as being most suited for a temperature stabilization of the frequency doubling element.

The advantages of a combination of a temperature stabilizing component with the frequency doubling element are as follows:
- Upon temperature stabilization, the performance of the frequency doubling element, in particular concerning linearity of the efficiency of frequency doubling and pulse stability of frequency-doubled light (typically green, 532 nm), within the whole required range of typically between 0.1 and 3 mJ, is generally improved.
- Particularly, due to stabilization of the temperature, an operator can select a maximum pulse energy of the SLT beam corresponding to an optical power density at the frequency doubling element only slightly below the destruction element, thus allowing to deliver a maximum power density to the eye.
- Surprisingly, due to such a temperature stabilization of the frequency doubling element, further beam attenuators, in the second and the third beam path, are not necessary.

With the use of only one beam attenuator means which is arranged within the first beam path the intensity of both treatment beams are controllable by the same means. Further the beam attenuator does not need to be of a particular expensive structure, as in cases of prior art where the beam attenuator is the switching element at the same time. At the same time the switching element between second and first beam path can be a very simple device like a mirror which is inserted or removed. For all of these reasons the invention allows a cost-effective and space-saving production, is less susceptible to failures and has a lower weight. Further the optical elements in the second and third beam path are less stressed by the laser energy from the laser source.

With respect to the wording that "any beam attenuation in the third beam path is solely controllable by said beam attenuator means in the first beam path" it should be clarified that this of course relates to the assembled state of the ophthalmic laser system being ready for use. The variable adjustment of the beam attenuator in the first beam path and thus of the beam attenuation in the third beam path allows the surgeon to variably control the total beam energy of the trabeculoplasty beam.

In order to account for the beam attenuation taking place upstream of the frequency doubling module it is necessary to improve the coupling of the beam into the frequency doubling module. This is achieved according to the invention by the beam shaping module upstream of said frequency doubling module.

The energy density of the beam should not decrease below a certain level, because the frequency conversion in an optical doubling medium is strongly dependent on the energy density of the beam. In a preferred embodiment the beam shaping module reduces the cross-section of the beam and thus increases its energy density. The combination of the beam attenuation within the first beam path and such a beam shaping module upstream of the frequency doubling module, comprising a temperature-stabilizing component, allows for an efficient and linear frequency conversion. Thus the advantages of space-saving, reliability and cost-effectiveness achieved by the invention do not adversely influence the beam conversion in the frequency doubler.

Further preferable embodiments are given in the Claims.

The invention will now be described in more detail and with respect to the accompanying drawings.
Fig. 1 shows an ophthalmic laser system according to the invention,
Fig. 2 shows the beam shaping module of the third beam path,
Fig. 3 shows the beam attenuator means of the first beam path.
Fig. 4 shows details of the combination of a temperature-stabilizing component with a frequency doubling element within a frequency doubling component.

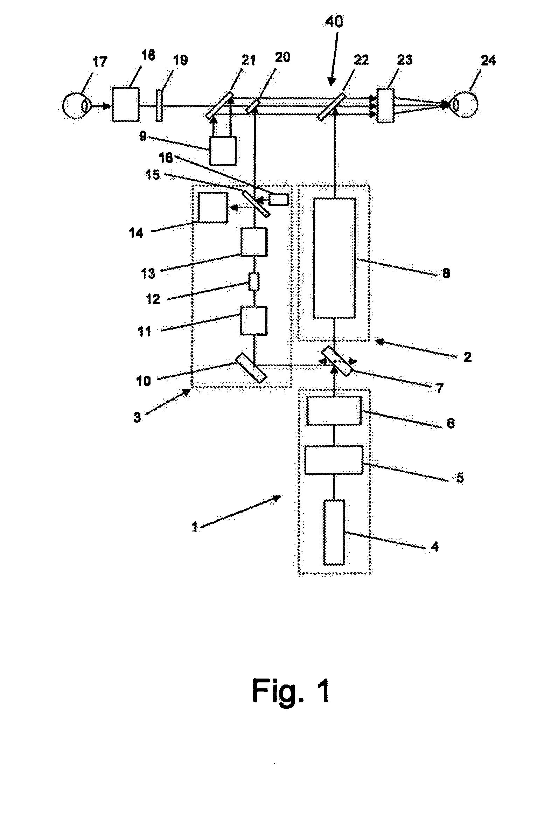

Fig. 1 shows an ophthalmic laser system comprising a laser module 4 producing a beam of short pulses of radiation with high energy density at a first wavelength, preferably of about 1064 nm. Such a wavelength is produced by a Nd:YAG laser. The laser module 4 is preferably a Q switched Nd : YAG laser operating in the infrared spectrum. However, other laser modules (such as Nd:YLF, Yb:YAG, Nd:YVO, etc) will also be suitable. A wavelength of 1064 nm, for example, allows photodisruption during treatment of secondary cataracts.

The laser module 4 is part of a first beam path 1. The beam of the first beam path 1 can be directed to a second beam path 2 or a third beam path 3 by operating an optical switching means 7. Preferably the optical switching means 7 is a mirror which is movable out of the beam path, thereby allowing the beam to be directed straight forward into the second beam path 2, or being arranged in the beam path, thereby deflecting the beam into the third beam path 3. Instead of a mirror also other switching elements as shutters, e.g. LCD shutters, or similar devices can be used.

A beam attenuator means 5 is arranged in the first beam path 1 between laser module 4 and optical switching means 7. The beam attenuator means 5 allows the regulation of the beam intensity in both operation modes, treatment of Glaucoma and treatment of secondary cataract. Fig. 3 shows an embodiment of the beam attenuator means 5 comprising two polarizing plates as functional parts being rotatable with respect to each other. In more detail the beam attenuator means 5 comprise a main housing 45, a fixed planar polarization plate 44, a rotating housing 43, a rotatable planar polarization plate 42 and a rotatable beam deviation compenstaion plate 41, the latter being arranged within the rotating housing 43. The reference sign 46 denotes the laser beam coming from the laser module 4.

The first beam path 1 further comprises an energy monitoring system 6 arranged between the beam attenuator means 5 and the optical switching means 7. The energy monitoring system 6 measures the energy of the laser pulses, in order to check the adjusted intensity and/or to determine the radiation exposure applied to the patient.

If the cataract treatment mode is selected and the beam is directed to the second beam path 2, the beam passes a beam shaping module 8 for adjusting the cross-section of the beam applied to the eye of the patient 24. Usually the beam shaping module 8 comprises a beam expander which widens the beam before guided into the objective lens, since the cross-section for secondary cataract treatment has to be smaller than for trabeculoplasty. The objective lens allows that way for better focusing of the laser beam.

At the end of the second beam path 2 means 22, 23 for directing the beam via a fourth beam path 40 into the eye 24 of a patient are provided. Preferably, the means 22 are comprised of a frequency sensitive or a broad band beam splitter allowing the deflection of a beam with a certain frequency or frequency band, respectively, and all other frequencies to pass without deflection. In this manner a frequency of e.g. 1064 nm of Nd:YAG laser will be deflected, whereas the visible range passes through the beam splitter without deflection, which is important for the observation of the patient's eye by a physician along the fourth beam path 40.

In the trabeculoplasty mode the beam is directed at the end of the first beam path 1 by the optical switching means 7 into the third beam path 3. The beam passes a deflection mirror 10, a first beam shaping module 11, a frequency doubling module 12, a second beam shaping module 13. The frequency doubling module 12 doubles the frequency of the laser beam coming from the first beam path 1. In the case of a 1064 nm laser the resulting frequency would amount to 532 nm, a wavelength in the green visible range and suitable to perform trabeculoplasty. The frequency doubling module 12 may comprise a non-linear optical crystal, for example a Potassium Titanyl Phosphate (KTP) doubling crystal.

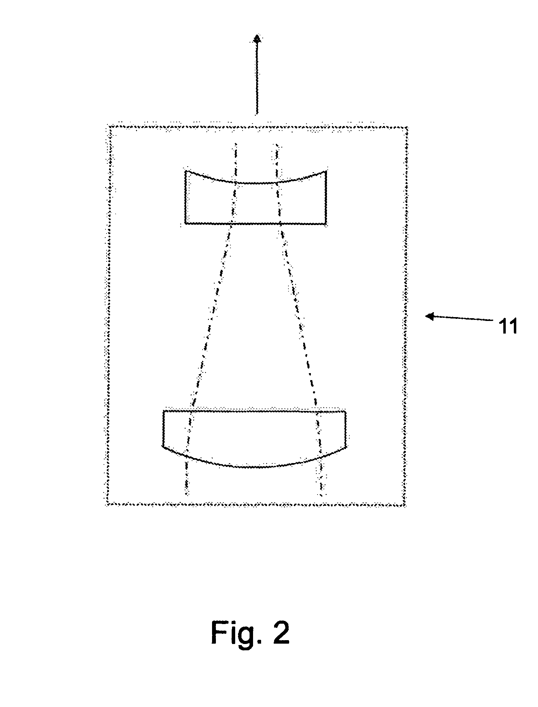

Fig. 2 shows an embodiment of the first beam shaping module 11. The parallel beam coming from the first beam path 1 passes a collecting lens and subsequently a dispersing lens. The lenses are configured to generate from a parallel incoming beam a parallel outgoing beam of smaller cross-section.

The arrow in Fig. 2 indicates the beam direction. As already mentioned above the arrangement of such a beam shaping module 11 before the frequency doubling module 12 constitutes a preferable embodiment. Due to the fact that the intensity regulation of the beam in the third beam path 3 is solely done by the beam attenuator means 5 in the first beam path 1 it is necessary - especially for low beam intensities - to condition the beam before entering the frequency doubling module 12 in order to achieve an efficient frequency doubling. This is done by decreasing the beam cross-section thereby increasing the energy density of the beam. Optionally, a second beam shaping module 13 is provided downstream of the frequency doubling module 12 for increasing or decreasing the beam diameter to a respective cross-section required/wanted for trabeculoplasty.

The third beam path 3 does not comprise a beam attenuator. Thus the intensity of the beam in the third beam path 3 is solely controllable by the beam attenuator means 5 of the first beam path 1. The advantage of such an arrangement consists in the fact, that both beams, trabeculoplasty beam and cataract beam, are controlled by the same attenuator. This is not only cost-effective but also space-saving and the control of the whole laser system is much easier. Also the energy monitoring system 6 can be used for both operation modes.

Optionally an additional energy monitoring system 14 is provided at the end of beam path 3 for checking the intensity of the laser pulses before being deflected towards the eye 24 of the patient. A beam splitter 15 deflects a minor amount of the beam towards the off-beam energy monitoring system 14. The same beam splitter 15 is used to feed a beam in the visible range by an aiming laser module 16 into the third beam path 3. The additional light is used for observation purposes by the physician. At the end of the third beam path 3 means 20 for directing the beam via a fourth beam path 40 into the eye 24 of a patient are provided. Preferably, the means 20 is a small mirror deflecting the trabeculoplasty beam of small diameter into the observation beam path 40. Because the dimensions of the mirror can be held small corresponding to the cross-section of the trabeculoplasty beam, it does not disturb the observation of the eye 24.

A separate laser aiming module 9 directs its beam towards a beam splitter 21 arranged within the fourth beam path 40. It is used for illuminating the eye of the patient exact at a position, where the treatment beam will be directed to. The aiming laser module 9 may comprise rotatable deflecting means for directing two or more narrow beams towards the beam splitter 21. The two or more narrow beams are arranged on the circumference of a virtual circle and rotate about an axis going through the center point of that circle. Such an arrangement is described in the US 4,499,897 A. A separation and rotation device is arranged downstream of the aiming laser and produced two beams for observation. The disclosure of the US 4,499,897 A is herewith incorporated into this specification - especially the drawings and the description of the drawings.

The observation beam path 40 comprises at the end facing to the physician optical observation elements 18 and filters 19 for protecting the surgeons eyes. A objective lens 23 for focusing the treatment beams and illumination beams towards the eye 24 of the patient is provided on the end facing to the patient. The trabeculoplasty beam coming from the third beam path 3 has a small cross-section and is therefore only marginally influenced by the objective lens. However, the cataract beam coming from the second beam path 2 has a larger cross-section and is thus focused by the objective lens to a very small cutting beam.

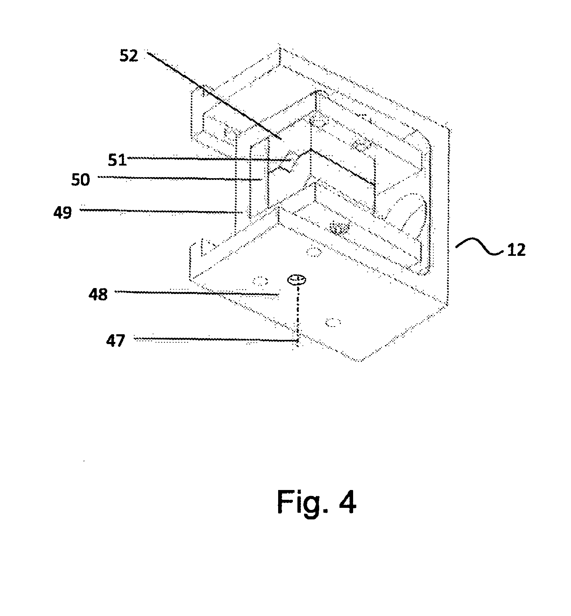

Fig. 4 shows details of the combination of a temperature-stabilizing component 50 with a frequency doubling element 51 within a frequency doubling component 12. The temperature stabilizing component can be a heating element. Preferably, the temperature-stabilizing component 50 is provided as a Peltier element, and the frequency doubling component 12 as a KTP doubling crystal.

Reference sign 48 denotes the main body of the frequency doubling component 12, 47 a rocking axis, 49 a rocking body, and 52 a holder for the frequency doubling element 51.

The temperature of the frequency doubling element 51 is stabilized by the temperature-stabilizing component 50 to which it is connected in thermal contact. Preferably the temperature of the frequency doubling element 51 is adjusted between 20°C and 40°C, most preferably between 27°C and 33°C. A temperature around 30°C has been verified as being most suited for a temperature stabilization of the frequency doubling element.

Thus, the performance of the frequency doubling element, in particular concerning linearity of the efficiency of frequency doubling and pulse stability of frequency-doubled light (typically green, 532 nm), within the whole required range of typically between 0.1 and 3 mJ, is generally improved.

### List of reference numerals

- 1: First beam path
- 2: Second beam path
- 3: Third beam path
- 4: Laser module
- 5: Beam attenuator means
- 6: Energy monitoring system
- 7: Optical switching means
- 8: Beam shaping module
- 9: Aiming laser module
- 10: Mirror
- 11: First beam shaping module
- 12: Frequency doubling module
- 13: Second beam shaping module
- 14: Energy monitoring system
- 15: Beam splitter
- 16: Aiming laser module
- 17: Operator's eye
- 18: Observation optic module
- 19: Safety filter module
- 20: Mirror
- 21: Beam splitter
- 22: Folding mirror
- 23: Objective lens
- 24: Patient's eye
- 40: Fourth beam path
- 41: Rotatable beam deviation compensation plate
- 42: Rotatable planar polarization plate
- 43: Rotating housing
- 44: Fixed planar polarization plate
- 45: Main housing of the beam attenuator
- 46: Beam from the laser module
- 47: Rocking axis
- 48: Main body
- 49: Rocking body
- 50: Temperature stabilizing element, particularly Peltier element
- 51: Frequency doubling element, particularly KTP doubling crystal
- 52: Holder for frequency doubling element

## Claims

1. An ophthalmic laser treatment system comprising:
a first beam path (1) incorporating a laser module (4) for producing a beam of short pulses of radiation with high energy density at a first wavelength and along a beam direction, and a beam attenuator means (5) downstream said laser module (4);
a second beam path (2);
a third beam path (3);
an optical switching means (7) allowing alternatively directing the beam from the first beam path (1) into the second beam path (2) or into the third beam path (3);
said second beam path (2) incorporating a beam expander for expanding said beam of short pulses at said first wavelength;
said third beam path (3) incorporating downstream of the optical switching means (7) a frequency doubling module (12), preferably comprising a non-linear crystal such as a KTP doubling crystal;
and means (20, 22, 23) for directing the beam of the second and third beam path (2, 3) into the eye (24) of a patient,
**characterized in, that**
the optical switching means (7) is arranged downstream of said beam attenuator means (5);
the third beam path (3) comprises a beam shaping module (11) upstream of said frequency doubling module (12);
the system lacks a further beam attenuator means and hence any beam attenuation in the third beam path (3) is solely controllable by said beam attenuator means (5) in the first beam path (1);
and the frequency doubling module (12) comprises a temperature stabilizing component (50) for stabilizing the temperature of a frequency doubling element (51) of the frequency doubling module (12) at a temperature around 30°C.

2. The ophthalmic laser treatment system of Claim 1, wherein the temperature stabilizing component (50) is provided as a heating element, particularly as a Peltier element, and/or for stabilizing the temperature of the frequency doubling element (51) between 20°C and 40°C, preferably between 27°C and 33°C, most preferably at 30°C.

3. The ophthalmic laser treatment system of Claim 1 or 2, wherein the third beam path (3) incorporates a second beam shaping module (13) arranged downstream of said frequency doubling module (12).

4. The ophthalmic laser treatment system according to one of Claims 1 to 3, wherein the beam shaping module (11) upstream of said frequency doubling module (12) reduces the cross-section of the beam coming from the laser module (4), and preferably generates from a parallel incoming beam a parallel outgoing beam.

5. The ophthalmic laser treatment system according to one of Claims 1 to 4, wherein the beam attenuator means (5) in the first beam path (1) is comprised of two polarizing plates being rotatable with respect to each another.

6. The ophthalmic laser treatment system according to one of Claims 1 to 5, wherein the first beam path (1) incorporates an energy monitoring system (6) arranged between the beam attenuator means (5) and the optical switching means (7).

7. The ophthalmic laser treatment system according to one of Claims 1 to 6, wherein the optical switching means (7) comprises a mirror being movable with respect to the beam paths (1, 2, 3).

8. The ophthalmic laser treatment system according to one of Claims 1 to 6, wherein the optical switching means (7) comprises a shutter, e.g. an LCD shutter.

9. The ophthalmic laser treatment system according to one of Claims 1 to 8 comprising a fourth beam path (40), wherein said means (20, 22, 23) for directing the beam of the second and third beam path (2, 3) into the eye (24) of a patient are arranged along the fourth beam path (40) which is adapted to allow for observation by a surgeon and preferably comprises respective optical observation elements (18) and filters (19) for protecting the surgeons eyes.

10. The ophthalmic laser treatment system according to one of Claims 1 to 9, wherein said means (20) for directing the beam of the third beam path (3) into the eye (24) of a patient is a mirror.

11. The ophthalmic laser treatment system according to one of Claims 1 to 10, wherein the means (22, 23) for directing the beam of the second beam path (2) into the eye (24) of a patient comprises at least one frequency sensitive beam splitter, preferably being sensitive for a frequency corresponding to a wavelength of 1064 nm.

12. The ophthalmic laser treatment system according to one of Claims 1 to 11, wherein the third beam path (3) comprises an energy monitoring system (14) for monitoring the laser energy within the third beam.

13. The ophthalmic laser treatment system according to one of Claims 1 to 12, wherein the third beam path (3) comprises an aiming laser module (16) for adding a laser beam in the visible range and in with a different frequency to the frequency of the beam coming from the frequency doubling module (12) and the beam shaping module (13) respectively and/or wherein the third beam path (3) comprises a beam splitter (15) downstream the frequency doubling module (12) and beam shaping means (13) respectively for adding the aiming beam coming from the laser aiming module (16).

14. The ophthalmic laser treatment system of Claim 13, wherein the same beam splitter (15) is configured for deflecting a fraction of the beam coming from the frequency doubling module (12) and the beam shaping means (13) respectively towards an energy monitoring system (14).

15. The ophthalmic laser treatment system according to one of Claims 1 to 14 wherein the laser module (4) comprises a solid state laser, preferably a Nd:YAG laser producing a beam at a wavelength of 1064 nm, and preferably flash lamp pumped.

## Patentansprüche

1. Augenlaserbehandlungssystem, umfassend:
einen ersten Strahlengang (1), der ein Lasermodul (4) zum Erzeugen eines Strahls von kurzen Strahlungspulsen mit hoher Energiedichte auf einer ersten Wellenlänge und entlang einer Strahlrichtung und ein Strahlabschwächungsmittel (5) nach dem Lasermodul (4) aufweist;
einen zweiten Strahlengang (2);
einen dritten Strahlengang (3);
ein optisches Umschaltmittel (7), das es erlaubt, den Strahl von dem ersten Strahlengang (1) wahlweise auf den zweiten Strahlengang (2) oder auf den dritten Strahlengang (3) umzulenken;
wobei der zweite Strahlengang (2) einen Strahlaufweiter aufweist, um den Strahl von kurzen Pulsen auf der ersten Wellenlänge aufzuweiten;
wobei der dritte Strahlengang (3) in Strahlausbreitungsrichtung nach dem optischen Umschaltmittel (7) ein Frequenzverdopplungsmodul (12) aufweist, das bevorzugt einen nichtlinearen Kristall wie etwa einen KTP-Verdopplungskristall umfasst; und
Mittel (20, 22, 23) zum Richten des Strahls des zweiten und dritten Strahlengangs (2, 3) auf das Auge (24) eines Patienten,
**dadurch gekennzeichnet, dass**
das optische Umschaltmittel (7) in Strahlausbreitungsrichtung nach dem Strahlabschwächungsmittel (5) angeordnet ist;
der dritte Strahlengang (3) ein Strahlformungsmodul (11) in Strahlausbreitungsrichtung vor dem Frequenzverdopplungsmodul (12) umfasst;
dem System ein weiteres Strahlabschwächungsmittel fehlt,
und daher eine eventuelle Strahlabschwächung in dem dritten Strahlengang (3) nur durch das Strahlenabschwächungsmittel (5) in dem ersten Strahlengang (1) steuerbar ist; und
das Frequenzverdopplungsmodul (12) eine Temperaturstabilisierungskomponente (50) umfasst, um die Temperatur eines Frequenzverdopplungselements (51) des Frequenzverdopplungsmoduls (12) auf einer Temperatur von ungefähr 30 °C zu stabilisieren.

2. Augenlaserbehandlungssystem nach Anspruch 1, wobei die Temperaturstabilisierungskomponente (50) als Heizelement, insbesondere als Peltier-Element, ausgeführt und/oder zum Stabilisieren der Temperatur des Frequenzverdopplungselements (51) zwischen 20 °C und 40 °C, bevorzugt zwischen 27 °C und 33 °C, höchst bevorzugt auf 30 °C ausgelegt ist.

3. Augenlaserbehandlungssystem nach Anspruch 1 oder 2, wobei der dritte Strahlengang (3) ein zweites Strahlformungsmodul (13) aufweist, das in Strahlausbreitungsrichtung nach dem Frequenzverdopplungsmodul (12) angeordnet ist.

4. Augenlaserbehandlungssystem nach einem der Ansprüche 1 bis 3, wobei das Strahlformungsmodul (11) in Strahlausbreitungsrichtung vor dem Frequenzverdopplungsmodul (12) den Querschnitt des Strahls, der aus dem Lasermodul (4) kommt, reduziert und bevorzugt aus einem parallelen einfallenden Strahl einen parallelen ausgehenden Strahl erzeugt.

5. Augenlaserbehandlungssystem nach einem der Ansprüche 1 bis 4, wobei das Strahlabschwächungsmittel (5) in dem ersten Strahlengang (1) aus zwei Polarisationsscheiben besteht, die relativ zueinander drehbar sind.

6. Augenlaserbehandlungssystem nach einem der Ansprüche 1 bis 5, wobei der erste Strahlengang (1) ein Energieüberwachungssystem (6) beinhaltet, das zwischen dem Strahlabschwächungsmittel (5) und dem optischen Umschaltmittel (7) angeordnet ist.

7. Augenlaserbehandlungssystem nach einem der Ansprüche 1 bis 6, wobei das optische Umschaltmittel (7) einen Spiegel umfasst, der relativ zu den Strahlengängen (1, 2, 3) bewegbar ist.

8. Augenlaserbehandlungssystem nach einem der Ansprüche 1 bis 6, wobei das optische Umschaltmittel (7) einen Verschluss, z.B. einen LCD-Verschluss, umfasst.

9. Augenlaserbehandlungssystem nach einem der Ansprüche 1 bis 8, umfassend einen vierten Strahlengang (40), wobei die Mittel (20, 22, 23) zum Richten des Strahls des zweiten und dritten Strahlengangs (2, 3) auf das Auge (24) eines Patienten entlang dem vierten Strahlengang (40) angeordnet sind, der geeignet ist, um die Beobachtung durch einen Chirurgen zu ermöglichen, und bevorzugt entsprechende optische Beobachtungselemente (18) und Filter (19) zum Schutz der Augen des Chirurgen umfasst.

10. Augenlaserbehandlungssystem nach einem der Ansprüche 1 bis 9, wobei das Mittel (20) zum Richten des Strahls des dritten Strahlengangs (3) auf das Auge (24) eines Patienten ein Spiegel ist.

11. Augenlaserbehandlungssystem nach einem der Ansprüche 1 bis 10, wobei das Mittel (22, 23) zum Richten des Strahls des zweiten Strahlengangs (2) auf das Auge (24) eines Patienten mindestens einen frequenzempfindlichen Strahlteiler umfasst, der bevorzugt auf eine Frequenz anspricht, die einer Wellenlänge von 1064 nm entspricht.

12. Augenlaserbehandlungssystem nach einem der Ansprüche 1 bis 11, wobei der dritte Strahlengang (3) ein Energieüberwachungssystem (14) zum Überwachen der Laserenergie in dem dritten Strahl umfasst.

13. Augenlaserbehandlungssystem nach einem der Ansprüche 1 bis 12, wobei der dritte Strahlengang (3) ein Pilotlasermodul (16) aufweist, um einen Laserstrahl im sichtbaren Bereich und mit einer anderen Frequenz als der Frequenz des Strahls, der aus dem Frequenzverdopplungsmodul (12) respektive dem Strahlformungsmodul (13) kommt, hinzuzufügen, und/oder wobei der dritte Strahlengang (3) einen Strahlenteiler (15) in Strahlausbreitungsrichtung nach dem Frequenzverdopplungsmodul (12) respektive dem Strahlformungsmittel (13) aufweist, zum Hinzufügen des aus dem Laserpilotmodul (16) kommenden Pilotlaserstrahls.

14. Augenlaserbehandlungssystem nach Anspruch 13, wobei der gleiche Strahlenteiler (15) konfiguriert ist, um einen Bruchteil des Strahls, der aus dem Frequenzverdopplungsmodul (12) respektive dem Strahlformungsmodul (13) kommt, in Richtung auf ein Energieüberwachungssystem (14) abzulenken.

15. Augenlaserbehandlungssystem nach einem der Ansprüche 1 bis 14, wobei das Lasermodul (4) einen Festkörperlaser, bevorzugt einen Nd:YAG-Laser, umfasst, der einen Strahl auf einer Wellenlänge von 1064 nm erzeugt, und der bevorzugt blitzlampengepumpt ist.

## Revendications

1. Système de traitement laser ophtalmique comprenant :
un premier trajet de faisceau (1) incorporant un module laser (4) pour produire un faisceau à courtes impulsions de radiation avec une haute densité d'énergie à une première longueur d'onde et le long d'une direction de faisceau, et un moyen d'atténuation de faisceau (5) en aval dudit module laser (4) ;
un deuxième trajet de faisceau (2) ;
un troisième trajet de faisceau (3) ;
un moyen de commutation optique (7) permettant de diriger le faisceau alternativement à partir du premier trajet de faisceau (1) jusqu'au deuxième trajet de faisceau (2) ou le troisième trajet de faisceau (3) ;
ledit deuxième trajet de faisceau (2) incorporant un élargisseur de faisceau pour élargir ledit faisceau à courtes impulsions à ladite première longueur d'onde ;
ledit troisième trajet de faisceau (3) incorporant, en aval du moyen de commutation optique (7), un module de doublage de fréquence (12) comprenant de préférence un cristal non linéaire, tel qu'un cristal doubleur KTP ;
et des moyens (20, 22, 23) pour diriger le faisceau des deuxième et troisième trajets de faisceau (2, 3) dans l'oeil (24) d'un patient, **caractérisé en ce que**
le moyen de commutation optique (7) est disposé en aval dudit moyen d'atténuation de faisceau (5) ;
le troisième trajet de faisceau (3) comprenant un module de conformation de faisceau (11) en amont dudit module de doublage de fréquence (12) ;
le système manquant d'un autre moyen d'atténuation de faisceau
et de ce fait, toute atténuation de faisceau dans le troisième trajet de faisceau (3) pouvant seulement être contrôlée grâce audit moyen d'atténuation de faisceau (5) dans le premier trajet de faisceau (1) ;
et le module de doublage de fréquence (12) comprenant un compostant de stabilisation de température (50) pour stabiliser la température d'un élément de doublage de fréquence (51) du module de doublage de fréquence (12) à une température autour de 30 °C.

2. Système de traitement laser ophtalmique selon la revendication 1, dans lequel le composant de stabilisation de température (50) est fourni en tant qu'élément chauffant, en particulier en tant qu'élément Peltier, et/ou pour stabiliser la température de l'élément de doublage de fréquence (51) entre 20 °C et 40 °C, de préférence entre 27 °C et 33 °C, de manière particulièrement préférée à 30 °C.

3. Système de traitement laser ophtalmique selon la revendication 1 ou 2, dans lequel le troisième trajet de faisceau (3) incorpore un deuxième module de conformation de faisceau (13) disposé en aval dudit module de doublage de fréquence (12).

4. Système de traitement laser ophtalmique selon l'une des revendications 1 à 3, dans lequel le module de conformation de faisceau (11) en amont dudit module de doublage de fréquence (12) réduit la section du faisceau provenant du module laser (4) et génère de préférence un faisceau sortant parallèle à partir d'un faisceau entrant parallèle.

5. Système de traitement laser ophtalmique selon l'une des revendications 1 à 4, dans lequel le moyen d'atténuation de faisceau (5) dans le premier trajet de faisceau (1) est formé par deux plaques de polarisation pouvant tourner l'une par rapport à l'autre.

6. Système de traitement laser ophtalmique selon l'une des revendications 1 à 5, dans lequel le premier trajet de faisceau (1) incorpore un système de surveillance d'énergie (6) disposé entre le moyen d'atténuation de faisceau (5) et le moyen de commutation optique (7).

7. Système de traitement laser ophtalmique selon l'une des revendications 1 à 6, dans lequel le moyen de commutation optique (7) comprend un miroir pouvant bouger par rapport aux trajets de faisceau (1, 2, 3).

8. Système de traitement laser ophtalmique selon l'une des revendications 1 à 6, dans lequel le moyen de commutation optique (7) comprend un obturateur, par exemple un obturateur LCD.

9. Système de traitement laser ophtalmique selon l'une des revendications 1 à 8, comprenant un quatrième trajet de faisceau (40), dans lequel lesdits moyens (20, 22, 23) pour diriger le faisceau des deuxième et troisième trajets de faisceau (2, 3) dans l'oeil (24) d'un patient sont disposés le long du quatrième trajet de faisceau (40) qui est adapté pour permettre une observation par un chirurgien et comprend de préférence des éléments d'observation optique (18) et des filtres (19) appropriés pour protéger les yeux du chirurgien.

10. Système de traitement laser ophtalmique selon l'une des revendications 1 à 9, dans lequel ledit moyen (20) pour diriger le faisceau du troisième trajet (3) dans l'oeil (24) d'un patient est un miroir.

11. Système de traitement laser ophtalmique selon l'une des revendications 1 à 10, dans lequel le moyen (22, 23) pour diriger le faisceau du deuxième trajet (2) dans l'oeil (24) d'un patient comprend au moins un séparateur de faisceau sensible à la fréquence, de préférence sensible à une fréquence correspondant à une longueur d'onde de 1064 nm.

12. Système de traitement laser ophtalmique selon l'une des revendications 1 à 11, dans lequel le troisième trajet de faisceau (3) comprend un système de surveillance d'énergie (14) pour surveiller l'énergie laser du troisième faisceau.

13. Système de traitement laser ophtalmique selon l'une des revendications 1 à 12, dans lequel le troisième trajet de faisceau (3) comprend un module de laser de visée (16) pour ajouter un faisceau laser dans la plage visible et avec une fréquence différente de la fréquence du faisceau provenant respectivement du module de doublage de fréquence (12) et du module de conformation de faisceau (13) et/ou, dans lequel, le troisième trajet de faisceau (3) comprend un séparateur de faisceau (15) respectivement en aval du module de doublage de fréquence (12) et du moyen de conformation de faisceau (13) afin d'ajouter le faisceau de visée provenant du module de faisceau de visée (16).

14. Système de traitement laser ophtalmique selon la revendication 13, dans lequel le même séparateur de faisceau (15) est configuré pour la déviation d'une fraction du faisceau provenant respectivement du module de doublage de fréquence (12) et du moyen de conformation de faisceau (13) vers un système de surveillance d'énergie (14).

15. Système de traitement laser ophtalmique selon l'une des revendications 1 à 14, dans lequel le module laser (4) comprend un laser solide, de préférence un laser Nd:YAG produisant un faisceau à une longueur d'onde de 1064 nm, et de préférence pompé par lampe éclair.
